(19) **Europäisches Patentamt / European Patent Office / Office européen des brevets**

(11) **EP 4 245 300 B1**

(12) **EUROPEAN PATENT SPECIFICATION**

(45) Date of publication and mention
of the grant of the patent:
**25.02.2026 Bulletin 2026/09**

(21) Application number: **22817859.6**

(22) Date of filing: **31.08.2022**

(51) International Patent Classification (IPC):
*A61K 9/24* (2006.01)    *A61K 9/28* (2006.01)
*A61K 31/167* (2006.01)    *A61K 47/02* (2006.01)
*A61K 47/12* (2006.01)    *A61K 47/34* (2017.01)
*A61P 25/04* (2006.01)

(52) Cooperative Patent Classification (CPC):
**A61P 25/04; A61K 9/209; A61K 9/28;
A61K 31/167; A61K 47/02; A61K 47/12;
A61K 47/34**

(86) International application number:
**PCT/JP2022/032859**

(87) International publication number:
**WO 2023/145120 (03.08.2023 Gazette 2023/31)**

(54) **CONTROLLED DRUG RELEASE PREPARATION COMPOSITION AND CONTROLLED DRUG RELEASE PREPARATION**

ZUBEREITUNGSZUSAMMENSETZUNG MIT KONTROLLIERTER WIRKSTOFFFREISETZUNG UND ZUBEREITUNG MIT KONTROLLIERTER WIRKSTOFFFREISETZUNG

COMPOSITION DE PRÉPARATION À LIBÉRATION CONTRÔLÉE DE MÉDICAMENT ET PRÉPARATION À LIBÉRATION CONTRÔLÉE DE MÉDICAMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.01.2022 JP 2022013780**

(43) Date of publication of application:
**20.09.2023 Bulletin 2023/38**

(73) Proprietor: **SUMITOMO SEIKA CHEMICALS CO., LTD.**
**Kako-gun, Hyogo 675-0145 (JP)**

(72) Inventors:
• **HACHIKEN, Shizuka**
**Hyogo (JP)**
• **IDO, Toru**
**Hyogo (JP)**

(74) Representative: **Vossius & Partner**
**Patentanwälte Rechtsanwälte mbB**
**Siebertstraße 3**
**81675 München (DE)**

(56) References cited:
| | |
|---|---|
| WO-A1-2021/029429 | WO-A1-2021/029429 |
| WO-A1-2023/145866 | WO-A2-97/37640 |
| CN-A- 106 727 406 | JP-A- 2000 508 313 |
| JP-A- 2021 516 260 | JP-A- S63 174 922 |
| US-A- 4 612 008 | US-A1- 2003 224 051 |

• **KAWAKITA KIMIO ET AL:** "Study on the Fundamental Properties of Powder Solids (I) - Correlations between Bulk Density and Particle Diameter, Fluidity or Angle of Repose, and between Particle Diameter and Fluidity -", JOURNAL OF THE JAPAN SOCIETY OF COLOUR MATERIAL, vol. 53, no. 6, 1 January 1980 (1980-01-01), pages 327 - 335, XP093082409
• **SAITO TOYOKAZU ET AL:** "27Q-pm001 On the dissolution characteristics of osmotic pump tablets.", ABSTRACTS OF THE ANNUAL MEETING OF THE PHARMACEUTICAL SOCIETY OF JAPAN., vol. 129, no. 4, 1 January 2009 (2009-01-01), XP093082078

**(Cont. next page)**

- **KAWAKITA KIMIO, YASUNOBU YUASA, MITSUO YAMASHIRO: "Study on the Fundamental Properties of Powder Solids (I) - Correlations between Bulk Density and Particle Diameter, Fluidity or Angle of Repose, and between Particle Diameter and Fluidity -", JOURNAL OF THE JAPAN SOCIETY OF COLOUR MATERIAL, vol. 53, no. 6, 1 January 1980 (1980-01-01), pages 327 - 335, XP093082409**

**Description**

Technical Field

The present invention relates to a controlled drug release preparation comprising a controlled drug release preparation composition.

Background Art

Conventionally, controlled drug release preparations are known to be able to control the drug release speed over a long period of time. As an example thereof, osmotic pump preparations have been proposed. The release rate of the drug from such an osmotic pump preparation is known to depend on the wall thickness (h) of the semipermeable membrane covering the preparation, wall permeability (K), effective drug concentration (C), preparation surface area (A), preparation radius (r), the volume of the pump layer in the preparation (Vp), the osmotic pressure gradient of the drug layer ($\Delta\pi d$), and the osmotic pressure gradient of the pump layer ($\Delta\pi p$). Specifically, the release rate of the drug substantially depends on the substance that forms the semipermeable membrane, wall thickness, the shape and size of the preparation, the type and amount of penetrant contained in the pump layer, the type and amount of polymer contained in the pump layer and drug layer, the type and amount of effective drug, and the composition ratio of the pump layer and drug layer (see, for example, PTL 1).

Some osmotic pump preparations are required to control the start of drug dissolution for the purpose of effective absorption of the drug in a specific affected area. In order to achieve this purpose, coating of preparations with enteric compositions etc. is conventionally known (see PTL 2). As another method, a special device for initially delayed delivery has been devised (see PTL 3). In a further dosage form for providing a substantially uniform drug release from a dosage form, the composition comprises a membrane comprising a semipermeable composition that surrounds a core comprising a drug layer comprising drug particles of 1 to 150 $\mu$m and polymer particles of 1 to 250 $\mu$m, and a displacement layer comprising an osmopolymer-hydrogel that imbibes fluid, hydrates and increases in swelling volume and thereby displaces the drug layer through an exit membrane selected from an exit in the group consisting of an orifice, passageway, pore, microporous channel, porous overlay, porous insert, micropore, microporous membrane and porepassageway (see PTL 4).

Citation List

Patent Literature

PTL 1: JPS60-41609A
PTL 2: JPS61-103820A
PTL 3: JPH06-505263A
PTL 4: WO 97/37640A2

Summary of Invention

Technical Problem

It is important for the controlled drug release preparations mentioned above to control the drug dissolution speed, particularly the dissolution start time; however, it has never been easy to control the dissolution speed. For example, it is possible to control the dissolution speed by adjusting the type and amount of drug contained in the controlled drug release preparations. However, since the type and amount of drug are generally specific to the intended preparation, and it has not been practical to control the dissolution speed by selecting the type and amount of drug. Another possible method of controlling the dissolution speed is to change the shape and size of the preparation; however, it is necessary to change the equipment used to obtain preparations; rather, there has also been a problem that it takes an enormous amount of time and money to finely adjust the dissolution speed.

On the other hand, it is also possible to finely adjust the dissolution speed by changing the semipermeable membrane-forming substance, the penetrant, and the type and amount of polymer contained in the pump layer and the drug layer. However, in this case, there is a cleaning process for the production equipment due to changes in the polymer type and blending amount, and it is necessary to conduct tests to confirm the dissolution speed many times. In addition, it may be necessary to change other processes, such as the granulation step, which is a typical step to obtain preparations.

Accordingly, the method of changing the type and amount of polymer requires an enormous amount of time and cost to establish an optimum production process, and the type of substance that can be practically selected is limited in terms of safety and price. Furthermore, it involves complicated work, such as application for change of substance (polymer type) and collection of information. For these reasons, this method is not an effective means. In particular, when it is desired to advance the dissolution start time of the drug, new preparation coating with enteric compositions etc. lengthens the process and increases the cost. Further, the use of special devices requires a more complicated preparation process, which is not a practical means for delaying the release start time.

The present invention was made in view of the above, and its object is to provide a controlled drug release preparation composition that can control the initial drug dissolution speed of a controlled drug release preparation by simple means, and the controlled drug release preparation. Solution to Problem

The present inventors conducted extensive research to achieve the above object, and consequently found that the above object can be achieved by polyalkylene oxide particles having specific particle size distribution. Thus, the present invention has been completed.

Specifically, the present invention provides a controlled drug release preparation comprising a controlled drug release preparation composition,

> the controlled drug release preparation having an osmotic pump structure with a core part and a coating layer coating the core part,
> the core part having a drug layer containing a drug, and a pump layer containing an osmotic agent,
> the coating layer being at least partially a semipermeable membrane,
> the coating layer having an opening for releasing the drug to the outside,
> the drug layer comprising a controlled drug release preparation composition,
> the controlled drug release preparation composition comprising polyalkylene oxide particles D,
> the polyalkylene oxide particles D comprising 40 mass% or less of polyalkylene oxide particles having a particle size of 300 $\mu$m or more,
> the pump layer comprising polyalkylene oxide particles P,
> the polyalkylene oxide particles P comprising 80 mass% or less of polyalkylene oxide particles having a particle size of 300 $\mu$m or more,
> wherein the content ratio of polyalkylene oxide particles having a particle size of 300 $\mu$m or more in polyalkylene oxide particles D or P is calculated by classifying polyalkylene oxide particles D or P by a sieve with a mesh opening of 300 $\mu$m according to JIS Z 8801-1, standard sieve, in accordance with the description.

According to further aspects of the invention, polyalkylene oxide particles D have a 5 mass% aqueous solution viscosity of 30 to 20000 mPa·s,polyalkylene oxide particles D have a bulk specific gravity of 0.35 to 0.60 g/mL,silica is contained in an amount of 1.5 parts by mass or less based on 100 parts by mass of the total amount of polyalkylene oxide particles D. In the afore controlled drug release preparation, polyalkylene oxide particles P can have a 0.5 mass% aqueous solution viscosity of 20 to 5000 mPa·s. Advantageous Effects of Invention

The controlled drug release preparation composition of the present invention can be suitably used for controlled drug release preparations, and can control the initial drug dissolution speed (in particular, the dissolution start time) by simple means. Further, the controlled drug release preparation of the present invention can easily control the drug dissolution start time.

Brief Description of Drawings

> Fig. 1 is a cross-sectional view schematically showing an embodiment of the controlled drug release preparation of the present invention.
> Fig. 2 shows changes over time in the drug dissolution rate of the preparations obtained in the Examples and Comparative Examples.
> Fig. 3 shows changes over time in the drug dissolution rate of the preparations obtained in the Examples and Comparative Examples.

Description of Embodiments

Embodiments of the present invention are described in detail below. In the present specification, the terms "comprising" and "containing" include the concepts of "comprising," "containing," "consisting essentially of," and "consisting of."

In the numerical range described in stages in the present specification, the upper or lower limit of the numerical range at one stage can be optionally combined with the upper or lower limit of the numerical range at another stage. In the numerical range described in the present specification, the upper or lower limit of the numerical range may be replaced with a value shown in the Examples or a value that can be uniquely derived from the Examples. Further, in the present specification, the numerical values connected by "to" mean the numerical range including the numerical values before and after "to" as the lower limit and the upper limit.

## 1. Controlled Drug Release Preparation Composition

The controlled drug release preparation composition of the present invention comprises polyalkylene oxide particles D, and polyalkylene oxide particles D comprise 40 mass% or less of polyalkylene oxide particles having a particle size of 300 $\mu$m or more. Because the controlled drug release preparation composition of the present invention comprises polyalkylene oxide particles D, when it is used for a controlled drug release preparation, the initial dissolution speed (also referred to as the release speed) of the drug can be easily controlled, and particularly the dissolution start time (also referred to as the release start time) of the drug can be advanced. Therefore, the controlled drug release preparation composition of the present invention can be suitably used for an osmotic pump controlled drug release preparation, and particularly suitably used in the drug layer of the controlled drug release preparation, described later. The osmotic pump controlled drug release preparation is described in detail in the "2. Controlled Drug Release Preparation" section.

Polyalkylene Oxide Particles D

Polyalkylene oxide particles D are obtained by forming polyalkylene oxide into particles, and comprise 40 mass% or less of polyalkylene oxide particles having a particle size of 300 $\mu$m or more, as described above. That is, the particle size distribution of polyalkylene oxide particles D is controlled in a specific range. The phrase "comprise 40 mass% or less of polyalkylene oxide particles having a particle size of 300 $\mu$m or more" can be read to mean that polyalkylene oxide particles having a particle size of 300 $\mu$m or more are not contained, or when polyalkylene oxide particles having a particle size of 300 $\mu$m or more are contained, the content ratio thereof is 40 mass% or less.

In polyalkylene oxide particles D, the type of polyalkylene oxide is not particularly limited, and examples include a wide range of known polyalkylene oxides.

In the polyalkylene oxide, the number of carbon atoms in the alkylene moiety is preferably 2 or more and 4 or less, for example. Because the effects of the present invention can be easily exhibited, it is particularly preferable that the alkylene moiety has 2 carbon atoms; that is, the polyalkylene oxide is preferably polyethylene oxide. Specific examples of polyalkylene oxides other than polyethylene oxide include polypropylene oxide, polybutylene oxide, ethylene oxide/propylene oxide copolymers, ethylene oxide/butylene oxide copolymers, and the like.

The polyalkylene oxide is generally a homopolymer, but is not limited thereto, and may be a copolymer. When the polyalkylene oxide is a copolymer, the polyalkylene oxide has, for example, two or more structural units with alkylene moieties having different number of carbon atoms.

Polyalkylene oxide particles D may contain one type of polyalkylene oxide, or two or more types of polyalkylene oxides.

Because the content ratio of polyalkylene oxide particles having a particle size of 300 $\mu$m or more is 40 mass% or less, when polyalkylene oxide particles D are used for a controlled drug release preparation (in particular, in the drug layer), it is possible to advance the initial dissolution start time of the drug. Polyalkylene oxide particles D preferably comprise polyalkylene oxide particles having a particle size of 300 $\mu$m or more in an amount of 35 mass% or less, more preferably 30 mass% or less, even more preferably 25 mass% or less, and particularly preferably 20 mass% or less. Polyalkylene oxide particles D preferably do not contain polyalkylene oxide particles having a particle size of 300 $\mu$m or more. From the production point of view, the content ratio of polyalkylene oxide particles having a particle size of 300 $\mu$m or more when contained may be, for example, 0.5 mass% or more.

In terms of ease of uniform mixing with a drug, polyalkylene oxide particles D preferably contain 80 mass% or less of polyalkylene oxide particles having a particle size of 180 $\mu$m or more. The content ratio of polyalkylene oxide particles having a particle size of 180 $\mu$m or more in polyalkylene oxide particles D may be 0. In terms of ease of production, the content ratio of such polyalkylene oxide particles may be 10 mass% or more, or 20 mass% or more.

The content ratio of polyalkylene oxide particles having a particle size of 300 $\mu$m or more in polyalkylene oxide particles D can be calculated by classifying polyalkylene oxide particles D by a sieve with a mesh opening of 300 $\mu$m (JIS Z 8801-1 standard sieve). Specifically, polyalkylene oxide particles D are classified by a sieve with a mesh opening of 300 $\mu$m, the mass of polyalkylene oxide particles remaining on the sieve is measured, and the ratio thereof based on the total mass of polyalkylene oxide particles D used in classification is calculated to thereby determine the content ratio of polyalkylene oxide particles having a particle size of 300 $\mu$m or more. As is clear from this explanation, the phrase "polyalkylene oxide particles having a particle size of 300 $\mu$m or more" refers to, after polyalkylene oxide particles D are classified by a sieve with a mesh opening of 300 $\mu$m, particles remaining on the sieve. Similarly, the phrase "polyalkylene oxide particles having a particle size of 180 $\mu$m or more" refers to, after polyalkylene oxide particles D are classified by a sieve with a mesh opening of 180 $\mu$m (JIS Z 8801-1 standard sieve), particles remaining on the sieve.

The method for adjusting the content ratio of polyalkylene oxide particles having a particle size of 300 $\mu$m or more in polyalkylene oxide particles D to 40 mass% or less is not particularly limited. For example, known particle size adjustment methods can be widely used. Examples of the adjustment method include a method for adjusting the particle size distribution in the process of producing polyalkylene oxide particles, and a method for adjusting the particle size distribution by classification or the like of the resulting polyalkylene oxide particles.

In an embodiment of the method for adjusting the particle size distribution, polyalkylene oxide particles obtained by a known production method or commercially available polyalkylene oxide particles are classified by a sieve with a mesh opening of 300 $\mu$m, particles remaining on the sieve and particles passing though the sieve are mixed at a prescribed ratio, thereby adjusting the content ratio of polyalkylene oxide particles having a particle size of 300 $\mu$m or more. The adjustment method according to the present embodiment can easily adjust the content ratio of polyalkylene oxide particles having a particle size of 300 $\mu$m or more to a desired range. In classification by a sieve with a mesh opening of 300 $\mu$m, only particles passing through the sieve can be used as polyalkylene oxide particles D.

Polyalkylene oxide particles D preferably have a 5 mass% aqueous solution viscosity of 30 to 20000 mPa·s. In this case, polyalkylene oxide particles D are likely to form a gel in the drug layer. The 5 mass% aqueous solution viscosity of polyalkylene oxide particles D is more preferably 40 mPa·s or more, and even more preferably 50 mPa·s or more, and is more preferably 10000 mPa·s or less, and even more preferably 1000 mPa·s or less. The 5 mass% aqueous solution viscosity of polyalkylene oxide particles D can be measured using a rotational viscometer (RV DVII+, produced by Brookfield).

Polyalkylene oxide particles D preferably have a bulk specific gravity of 0.35 to 0.60 g/mL. In this case, production efficiency and transport efficiency are excellent, and the formability of the preparation tends to be good. The bulk specific gravity of polyalkylene oxide particles D is more preferably 0.35 to 0.55 g/mL.

Polyalkylene oxide particles D preferably have a mass average molecular weight of 100,000 or more in terms of ease of forming a gel in the drug layer, and preferably have a mass average molecular weight of 1 million or less in terms of ease of obtaining a soft gel. The mass average molecular weight of polyalkylene oxide particles D is more preferably 150,000 to 800,000, and even more preferably 150,000 to 600,000. The mass average molecular weight of polyalkylene oxide particles D as mentioned herein refers to a value measured by gel permeation chromatography, and particularly refers to a value calculated from a calibration curve created using a known polyethylene oxide standard sample.

The method for producing polyalkylene oxide particles D is not particularly limited. For example, methods for producing known polyalkylene oxide particles can be also widely used in the present invention. For example, polyalkylene oxide particles D can be obtained by polymerization reaction of an alkylene oxide in the presence of an alkali or a metal catalyst. Examples of the alkylene oxide used herein include aliphatic alkylene oxides. Specific examples include ethylene oxide, propylene oxide, and butylene oxide; preferably ethylene oxide or propylene oxide; and particularly preferably ethylene oxide. Alkylene oxides can be used singly or in combination of two or more.

As the metal catalyst, for example, metal catalysts conventionally used in the production of polyalkylene oxide can be widely used. Of these, an organic zinc catalyst is preferred. Organic zinc catalysts can be obtained by known production methods, preferably by reacting organic zinc compounds with aliphatic polyhydric alcohols and monohydric alcohols to form particulate reaction products.

The metal catalyst can be used in a catalytic amount, and is preferably used in an amount of 0.00005 mol or more per mol of alkylene oxide. Because the amount of catalyst used is within this range, the decrease in the polymerization reaction rate is easily suppressed, and the polymerization time can be controlled to be shorter.

The polymerization reaction of the alkylene oxide can be performed in a solvent. As such solvents, those used in methods for producing known polyalkylene oxides can be widely used. For example, at least one hydrocarbon solvent selected from the group consisting of n-pentane, n-hexane, n-heptane, and cyclohexane can be used. N-hexane or n-pentane is preferably used because they are easily available industrially, and because they have a boiling point lower than the melting point of the resulting polyalkylene oxide and are easy to remove after the polymerization reaction. The amount of polymerization solvent used is preferably 200 to 10000 parts by mass, and more preferably 400 to 600 parts by mass, based on 100 parts by mass of alkylene oxide, in terms of removing the heat of polymerization and easily controlling the polymerization reaction.

A chain transfer agent can be used in the polymerization reaction of the alkylene oxide. This can easily adjust the molecular weight of the polyalkylene oxide. The type of chain transfer agent is not particularly limited, and known chain transfer agents that can be used in the polymerization reaction of alkylene oxides can be widely used. Examples of chain transfer agents include $C_{1-5}$ alcohol compounds, such as methanol, ethanol, n-propanol, isopropanol, n-butanol, isobutanol, and t-butanol.

When a chain transfer agent is used, the amount thereof used is not particularly limited, and can be, for example, 0.002 to 0.3 mol% based on the alkylene oxide.

The temperature and other conditions of the polymerization reaction of the alkylene oxide are not particularly limited, and can be the same as known conditions.

The polyalkylene oxide obtained by the above polymerization reaction can be irradiated with active energy rays, if necessary. As a result, the polyalkylene oxide is decomposed, and the molecular weight can be adjusted within a desired range. Examples of active energy rays include radiation such as gamma rays, ultraviolet rays, X-rays, ion beam, and the like. In terms of the ease of adjusting the molecular weight, gamma rays are preferred.

Controlled Drug Release Preparation Composition

The controlled drug release preparation composition of the present invention may consist of polyalkylene oxide particles D or may comprise components other than polyalkylene oxide particles D. Examples of components other than polyalkylene oxide particles D include silica, as well as various known additives.

When the controlled drug release preparation composition of the present invention contains polyalkylene oxide particles D and silica, silica is preferably contained in an amount of 1.5 parts by mass or less based on 100 parts by mass of the total amount of polyalkylene oxide particles D. In this case, silica adheres to the surface of polyalkylene oxide particles D and is likely to be present, and the fluidity of polyalkylene oxide particles D is likely to be improved. From the viewpoint that silica is likely to uniformly adhere to the particle surface, the content of silica based on 100 parts by mass of polyalkylene oxide particles D is more preferably 1.3 parts by mass or less, and even more preferably 1.2 parts by mass or less, and is more preferably 0.2 parts by mass or more, and even more preferably 0.5 parts by mass or more. The controlled drug release preparation composition of the present invention may consist of polyalkylene oxide particles D and silica. Polyalkylene oxide particles D may not contain silica.

The type of silica is not particularly limited. For example, silica conventionally used for the purpose of improving the fluidity of polyalkylene oxide particles in controlled drug release preparations can be widely used.

2. Controlled Drug Release Preparation

The present invention also includes a controlled drug release preparation. In an embodiment of the controlled drug release preparation, the invention comprises the controlled drug release preparation composition described above, and has an osmotic pump structure with a core part and a coating layer coating the core part, wherein the core part has a drug layer containing a drug and a pump layer containing an osmotic agent, the coating layer is at least partially a semipermeable membrane, the coating layer has an opening for releasing the drug to the outside, and the drug layer comprises the controlled drug release preparation composition.

Since the controlled drug release preparation has an osmotic pump structure with a core part and a coating layer, as described above, when it is administered into the patient's body, water or body fluid permeates into the core part through the semipermeable membrane, the pump layer is swollen due to osmotic pressure, and the drug can be eluted from the opening provided in the coating layer.

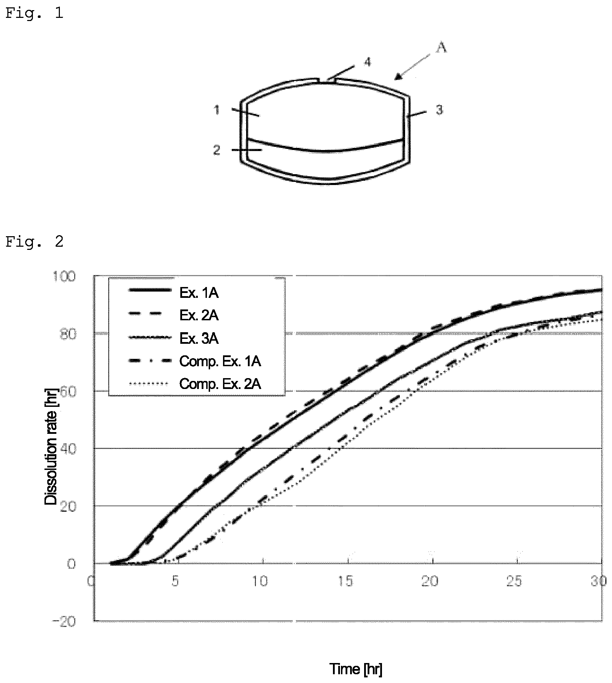

Fig. 1 is a cross-sectional view schematically showing an embodiment of the controlled drug release preparation. In a controlled drug release preparation A shown in Fig. 1, a drug layer 1 and a pump layer 2 are laminated in this order to form a core part, and the core part is coated with a coating layer 3. The coating layer 3 is provided with an opening 4. The controlled drug release preparation A may have a structure other than the structure shown in Fig. 1 as long as it has an osmotic pump structure with a core part and a coating layer. For example, structures of known osmotic pump controlled drug release preparations can be widely used.

Drug Layer

In the controlled drug release preparation of the present invention, the drug layer contains a drug and the controlled drug release preparation composition. Therefore, the drug layer contains a drug and polyalkylene oxide particles D (containing 40 mass% or less of polyalkylene oxide particles having a particle size of 300 μm or more) as essential components.

The content ratio of polyalkylene oxide particles D contained in the drug layer can be 40 mass% or more and 95 mass% or less based on the total mass of the drug layer. The content ratio of polyalkylene oxide particles D contained in the drug layer is preferably 50 mass% or more, and more preferably 60 mass% or more, and is preferably 90 mass% or less, and more preferably 85 mass% or less.

Examples of the drug contained in the drug layer include a wide range of various drugs intended for sustained release. For example, drugs applied to known controlled drug release preparations can be also widely applied to the present invention. The drug contained in the drug layer can be a single drug or a mixture of two or more drugs. Examples of drugs include acetaminophen used as an antipyretic and analgesic.

The content ratio of the drug contained in the drug layer can be 5 mass% or more and 60 mass% or less based on the total mass of the drug layer. The content ratio of the drug contained in the drug layer is preferably 10 mass% or more, and more preferably 15 mass% or more, and is preferably 50 mass% or less, and more preferably 40 mass% or less.

The method for forming the drug layer is not particularly limited. For example, methods for forming drug layers used for known controlled drug release preparations can be also widely used in the present invention.

The drug layer may contain, if necessary, additives such as diluents, excipients, lubricants, dyes, and pigments, in addition to polyalkylene oxide particles D. As such additives, those used for conventional controlled drug release preparations can be widely used.

Pump Layer

The pump layer contains an osmotic agent. Examples of osmotic agents include substances that do not adversely affect preparations. For example, osmotic agents applied to known controlled drug release preparations can be also widely applied to the present invention. Specifically, water-soluble substances that can be used as additives for medicines can be used. Examples include inorganic salts, water-soluble salts of organic acids, water-soluble nonionic organic substances, and the like.

Examples of inorganic salts include chlorides of alkali metals or alkaline earth metals, such as lithium, sodium, potassium, magnesium, and calcium; sulfate, carbonate, bicarbonate, phosphate, hydrogen or dihydrogen phosphate, and the like. Examples of water-soluble salts of organic acids include acetate, succinate, benzoate, citric acid, ascorbate, and the like. Examples of water-soluble nonionic organic substances include carbohydrates, such as sugars and amino acids. Of these, sodium chloride is preferably used in terms of ease of handling and low cost. The pump layer may contain a single osmotic agent or two or more osmotic agents.

The content ratio of the osmotic agent can be 10 mass% or more and 55 mass% or less based on the total mass of the pump layer. The content ratio of the osmotic agent contained in the pump layer is preferably 15 mass% or more, and more preferably 20 mass% or more, and is preferably 45 mass% or less, and more preferably 40 mass% or less.

The pump layer may contain polyalkylene oxide particles in addition to the osmotic agent. Because the pump layer contains polyalkylene oxide particles, the controlled drug release preparation is likely to allow a constant speed of drug release (zero-order release).

The polyalkylene oxide particles contained in the pump layer may be polyalkylene oxide particles D described above, or may be polyalkylene oxide particles other than polyalkylene oxide particles D; polyalkylene oxide particles other than polyalkylene oxide particles D are preferred. Hereinafter, the polyalkylene oxide particles contained in the pump layer are abbreviated as "polyalkylene oxide particles P."

Polyalkylene oxide particles P may be polyalkylene oxide particles with a particle size distribution different from polyalkylene oxide particles D. Polyalkylene oxide particles P may have the same composition as that of polyalkylene oxide particles D. Polyalkylene oxide particles P are also preferably polyethylene oxide particles, as with polyalkylene oxide particles D.

The particle size of polyalkylene oxide particles P is not particularly limited; however, in terms of further advancing the dissolution start time of the drug, polyalkylene oxide particles P preferably comprise 80 mass% or less of polyalkylene oxide particles having a particle size of 300 $\mu$m or more. In summary, it is preferable for the controlled drug release preparation of the present invention that the pump layer comprises polyalkylene oxide particles P, and that polyalkylene oxide particles P comprise 80 mass% or less of polyalkylene oxide particles having a particle size of 300 $\mu$m or more. The phrase "comprise 80 mass% or less of polyalkylene oxide particles having a particle size of 300 $\mu$m or more" can be read to mean that polyalkylene oxide particles having a particle size of 300 $\mu$m or more are not contained, or when polyalkylene oxide particles having a particle size of 300 $\mu$m or more are contained, the content ratio thereof is 80 mass% or less.

Polyalkylene oxide particles P preferably comprise polyalkylene oxide particles having a particle size of 300 $\mu$m or more in an amount of 70 mass% or less, more preferably 40 mass% or less, and particularly preferably 20 mass% or less. For example, polyalkylene oxide particles P may contain 5 to 20 mass% of polyalkylene oxide particles having a particle size of 300 $\mu$m or more.

As the method for adjusting the particle size distribution of polyalkylene oxide particles P, the same method for polyalkylene oxide particles D can also be used. Specifically, in a preferred method, the particle size distribution can be adjusted by the polymerization conditions for producing polyalkylene oxide particles, or polyalkylene oxide particles are classified by a sieve with a mesh opening of 300 $\mu$m, and particles remaining on the sieve and particles passing through the sieve are mixed at a prescribed ratio.

Polyalkylene oxide particles P preferably have a 0.5 mass% aqueous solution viscosity of 20 to 5000 mPa·s, in terms of industrial availability. The 0.5 mass% aqueous solution viscosity of polyalkylene oxide particles P is more preferably 70 mPa·s or more, and even more preferably 100 mPa·s or more, and is more preferably 2000 mPa·s or less, even more preferably 1500 mPa·s or less, and particularly preferably 1000 mPa·s or less. The 0.5 mass% aqueous solution viscosity of polyalkylene oxide particles P can be measured using a rotational viscometer (RV DVII+, produced by Brookfield).

Polyalkylene oxide particles P preferably have a bulk specific gravity of 0.35 to 0.60 g/mL. In this case, production efficiency and transport efficiency are excellent, and the formability of the preparation tends to be good. The bulk specific gravity of polyalkylene oxide particles P is more preferably 0.35 to 0.55 g/mL.

Polyalkylene oxide particles P may contain silica for the purpose of improving fluidity, as with polyalkylene oxide particles D. When polyalkylene oxide particles P contain silica, silica is preferably contained in an amount of 1.5 parts by mass or less based on 100 parts by mass of the total amount of polyalkylene oxide particles P. In this case, silica adheres to the surface of polyalkylene oxide particles P and is likely to be present, and the fluidity of polyalkylene oxide particles P is likely to be improved. From the viewpoint that silica is likely to uniformly adhere to the particle surface, the content of silica based on 100 parts by mass of polyalkylene oxide particles P is more preferably 1.3 parts by mass or less, and even more preferably 1.2 parts by mass or less, and is more preferably 0.2 parts by mass or more, and even more preferably 0.5 parts by mass or more. The type of silica is also not particularly limited. For example, it is possible to widely use silica that has been used for the purpose of improving the fluidity of polyalkylene oxide particles in controlled drug release preparations.

Polyalkylene oxide particles P preferably have a mass average molecular weight of 1 million or more in terms of ease of forming a gel in the pump layer, and preferably have a mass average molecular weight of 15 million or less in terms of ease of obtaining a soft gel. The mass average molecular weight of polyalkylene oxide particles P is more preferably 2 million to 12 million, and even more preferably 3 million to 10 million. The mass average molecular weight of polyalkylene oxide particles P as mentioned herein refers to a value measured by gel permeation chromatography, and particularly refers to a value calculated from a calibration curve created using a known polyethylene oxide standard sample.

The method for producing polyalkylene oxide particles P is not particularly limited, and the same method as the method for producing polyalkylene oxide particles D can be used.

The content ratio of polyalkylene oxide particles P contained in the pump layer can be 45 mass% or more and 90 mass% or less based on the total mass of the pump layer. The content ratio of polyalkylene oxide particles P contained in the pump layer is preferably 55 mass% or more, and more preferably 60 mass% or more, and is preferably 80 mass% or less, and more preferably 75 mass% or less.

The method for forming the pump layer is not particularly limited. For example, methods for producing pump layers used for known controlled drug release preparations can be also widely used in the present invention.

The pump layer may contain, if necessary, additives such as diluents, excipients, lubricants, dyes, and pigments, in addition to polyalkylene oxide particles P. As such additives, those used for conventional controlled drug release preparations can be widely used.

Core Part

In the core part, the mass ratio of the drug layer to the pump layer (drug layer/pump layer) is preferably, for example, 1 to 5, and more preferably 2 to 3.

The core part can be formed by laminating the drug layer and the pump layer by a known method such as compression molding. For example, it can be formed into a double-layered tablet shown in Fig. 1, as described above.

The size of the core part varies depending on the intended use of the preparation etc., and thus cannot be determined unconditionally. For example, the size of the core part can be about 5 to 12 mm in diameter and about 3 to 7 mm in thickness. In this case, it is suitable for tablets to be orally administered.

Coating Layer

The core part is coated with a coating layer. The coating layer can be formed with a pharmaceutically acceptable material. The coating layer is, for example, at least partially or wholly a semipermeable membrane. The ratio of the semipermeable membrane in the coating layer is not particularly limited as long as the core part is coated with the semipermeable membrane to the extent that permeation of water or body fluid is not impaired. In terms of easy coating of the core part, the entire coating layer is made of a semipermeable membrane; that is, the core part is preferably coated with a semipermeable membrane.

Semipermeable membranes are permeable to water but impermeable to drugs and osmotic agents. The material for forming such a semipermeable membrane is preferably a known polymer substance or the like that is not metabolized in the gastrointestinal tract. From this point of view, examples of the material for forming the semipermeable membrane include cellulose acetate and other cellulose acetate derivatives, such as cellulose acetate, triacetyl cellulose, cellulose acetate ethyl carbamate, cellulose acetate methyl sulfonate, and cellulose acetate diethylaminoacetate; poly(vinyl methyl ether) copolymers; selectively permeable aromatic nitrogen compounds containing a polymeric membrane that exhibits water permeability and little solute permeability; polymeric epoxides; substances made from copolymers of alkene oxides and alkyl glycidyl ethers; semipermeable corrosive polyglycolic acid; and the like. Of these, cellulose acetate is preferably used in terms of ease of handling and low cost.

Further, the semipermeable membrane may contain, if necessary, plasticizers such as polyethylene glycol;

substances that increase the porosity of the semipermeable membrane, such as sucrose, lactose, sodium chloride, and potassium chloride; substances for shading, such as titanium dioxide and iron oxide; and the like.

The method for forming the coating layer is not particularly limited. For example, known methods can be widely used. For example, the coating layer can be formed by spray-coating the core part with a substance for forming the coating layer, preferably a solution of the material for forming the semipermeable membrane.

The thickness of the coating layer differs depending on the size of the preparation etc., and is thus not particularly limited. The thickness of the coating layer can be appropriately designed in consideration of the permeation speed of water or body fluid into the core part.

In the controlled drug release preparation of the present invention, the coating layer may have an opening (see opening 4 in Fig. 1). Forming an opening facilitates the release of the drug to the outside through the opening. The coating layer has at least one opening.

The opening can be provided in the coating layer after the core part having a drug layer containing a drug and a pump layer containing an osmotic agent is coated with the coating layer. The opening can be provided in the coating layer, for example, by forming a hole with a laser, mechanically forming a hole, or forming a hole by punching or the like. Methods known in this technical field can be widely used.

The size of the opening can be set in a suitable range depending on the size of the preparation etc. For example, the diameter is preferably 0.2 to 2 mm, and more preferably 0.5 to 1.5 mm.

Controlled Drug Release Preparation

Because the controlled drug release preparation of the present invention is configured as described above, when the controlled drug release preparation is administered to the patient's body, the patient's body fluid permeates the coating layer and swells the pump layer. When the pump layer is swollen, the drug layer is pushed in the direction of the opening, and the drug contained in the drug layer is released, for example, from the opening.

In particular, in the controlled drug release preparation of the present invention, the drug layer contains polyalkylene oxide particles D, and polyalkylene oxide particles D contained in the drug layer are swollen by absorbing water penetrating into the preparation through the semipermeable membrane, thereby extruding the drug from the opening. Although a restrictive interpretation is not necessarily desired, it is presumed that due to the specific particle size distribution of polyalkylene oxide particles D, the drug layer is swollen faster, which can consequently advance the dissolution start time of the drug and accelerate the initial dissolution speed of the drug.

Conventionally, polyalkylene oxides used in this type of preparation contain more than 40 mass% of polyalkylene oxide particles having a particle size of 300 $\mu$m or more, and it is thus difficult to accelerate the dissolution start time of the drug; however, the drug layer containing polyalkylene oxide particles D facilitates the adjustment of the dissolution start time of the drug.

The controlled drug release preparation of the present invention can accelerate the dissolution start time, specifically, the time to reach a drug dissolution rate of 5% and 10%, compared with before. For example, the time to reach a drug dissolution rate of 5% can be within 5 hours (preferably within 4 hours), and the time to reach a drug dissolution rate of 10% can be within 7 hours (preferably within 6 hours).

As described above, the controlled drug release preparation of the present invention can make the induction period until the start of drug dissolution shorter than before.

The controlled drug release preparation of the present invention can easily adjust the drug dissolution start time by adjusting the particle size of polyalkylene oxide mixed in the drug layer, without changing the size of the preparation, the semipermeable membrane, the drug, the osmotic agent, and the type and amount of polymer, and without using new coating materials, such as enteric compositions, or special devices.

Moreover, the use of the controlled drug release preparation of the present invention can reduce the frequency of administration to the patient. As a result, it is also expected to reduce the burden on the patient. That is, the controlled drug release preparation of the present invention with controlled drug dissolution speed can be administered once or twice a day, whereby the active ingredient can be released into the body substantially uniformly over a long period of time. In the case of administration once a day, the drug dissolution rate after 24 hours is preferably 80% or more.

The controlled drug release preparation of the present invention can be used for, for example, various antidepressants, antihypertensive agents, urinary system disease therapeutic agents, anticancer agents, and the like.

The method for producing the controlled drug release preparation of the present invention is not particularly limited. For example, methods for producing known controlled drug release preparations can be widely used. For example, the production of each layer may include a granulation step, and different solvents may be used for granulation. Further, if necessary, the material for forming each layer can be mixed with additives such as diluents, excipients, lubricants, dyes, and pigments. As such additives, those used for conventional controlled drug release preparations can be widely used.

The controlled drug release preparation of the present invention may be in a form other than the form shown in Fig. 1. For example, other layers, such as a delay layer for delaying the start of drug dissolution, may be present between the drug layer and the opening, or other layers, such as a lubricating layer for facilitating drug release, may be present between the core part and the coating layer.

The controlled drug release preparation of the present invention can be applied to various osmotic pump preparations within a range not departing from the gist of the present invention.

Examples

The present invention is described in more detail below with reference to Examples; however, the present invention is not limited to these Examples.

Production Example 1

Diethyl zinc was diluted with n-hexane to a concentration of 0.66 mol/L in terms of zinc in a vessel purged with nitrogen. The vessel was then cooled to 10°C, and 1,4-butanediol was added to the vessel under stirring over 9 hours until the concentrations of 1,4-butanediol and ethanol reached 0.59 mol/L and 4.29 mol/L, respectively, with respect to n-hexane. After the completion of the addition, the temperature in the vessel was raised to 30°C, and diethyl zinc was reacted with 1,4-butanediol and ethanol for 1 hour. Next, the temperature was raised to 50°C, and the reaction was performed for 1 hour. The temperature in the vessel was then raised to 130°C, and the unreacted alcohol was removed from the system together with n-hexane. After cooling, the reaction liquid in the vessel was diluted with n-hexane so that the concentration of the organic zinc catalyst was 3 mass%, thereby obtaining a dispersion containing the organic zinc catalyst.

Production Example 2

The dispersion obtained in Production Example 1 was mixed with n-hexane in a pressure-resistant vessel purged with nitrogen to prepare an n-hexane solution with a concentration of 0.0016 mol/L of organic zinc catalyst in terms of zinc. To the n-hexane solution, ethylene oxide was added to a concentration of 3.49 mol/L, the vessel was sealed, and the mixture was polymerized with stirring in a thermostatic bath at 40°C for 6 hours. After the completion of the polymerization, the produced white product was filtered out and dried under reduced pressure at 40°C. The resulting dried particles were mixed with 1 mass% of amorphous silica (Aerosil, produced by Nippon Aerosil Co., Ltd.) to obtain silica-attached polyethylene oxide particles (hereinafter referred to as "polyethylene oxide particles 1"). The 0.5 mass% aqueous solution viscosity of polyethylene oxide particles 1 was 385 mPa·s.

Production Example 3

Silica-attached polyethylene oxide particles 1 obtained in Production Example 2 were irradiated with gamma rays, thereby obtaining polyethylene oxide particles having a 5 mass% aqueous solution viscosity of 180 mPa·s (hereinafter referred to as "polyethylene oxide particles 2").

The 5 mass% aqueous solution viscosity and 0.5 mass% aqueous solution viscosity of the polyethylene oxide particles were measured as described below.

5 Mass% Aqueous Solution Viscosity of Polyethylene Oxide Particles

30 g of the polyethylene oxide particles and 125 mL of isopropanol were added to a 1-L beaker, and while stirring at 300 to 400 rpm using an impeller, 570 g of ion-exchange water was added, and the mixture was stirred for 1 minute. Then, the stirring speed was changed to 60 rpm, and stirring was further continued for 3 hours, thereby obtaining a 5 mass% aqueous solution of the polyethylene oxide particles. The aqueous solution was maintained at 25°C, the viscosity was measured using a rotational viscometer (RV DVII+, produced by Brookfield), and this value was taken as the 5 mass% aqueous solution viscosity.

0.5 Mass% Aqueous Solution Viscosity of Polyethylene Oxide Particles

3 g of the polyethylene oxide particles and 125 mL of isopropanol were added to a 1-L beaker, and while stirring at 300 to 400 rpm using an impeller, 597 g of ion-exchange water was added, and the mixture was stirred for 1 minute. Then, the stirring speed was changed to 60 rpm, and stirring was further continued for 3 hours, thereby obtaining a 0.5 mass% aqueous solution of the polyethylene oxide particles. The aqueous solution was maintained at 25°C, the viscosity was measured using a rotational viscometer (RV DVII+, produced by Brookfield), and this value was taken as the 0.5 mass% aqueous solution viscosity.

Example 1

Polyethylene oxide particles 2 obtained in Production Example 3 were classified by a sieve with a mesh opening of 300 $\mu$m, and particles on the sieve and particles below the sieve (particles passing through the sieve) were mixed at a mass ratio (above sieve/below sieve) of 14/86. As a result, polyethylene oxide particles (D-1) containing 14 mass% of polyethylene oxide particles having a particle size of 300 $\mu$m or more were obtained.

Example 2

Polyethylene oxide particles 2 obtained in Production Example 3 were classified by a sieve with a mesh opening of 300 $\mu$m, and particles on the sieve and particles below the sieve (particles passing through the sieve) were mixed at a mass ratio (above sieve/below sieve) of 2/98. As a result, polyethylene oxide particles (D-2) containing 2 mass% of polyethylene oxide particles having a particle size of 300 $\mu$m or more were obtained.

Example 3

Polyethylene oxide particles 2 obtained in Production Example 3 were classified by a sieve with a mesh opening of 300 $\mu$m, and particles on the sieve and particles below the sieve (particles passing through the sieve) were mixed at a mass ratio (above sieve/below sieve) of 32/68. As a result, polyethylene oxide particles (D-3) containing 32 mass% of polyethylene oxide particles having a particle size of 300 $\mu$m or more were obtained.

Comparative Example 1

Polyethylene oxide particles 2 obtained in Production Example 3 were classified by a sieve with a mesh opening of 300 $\mu$m, and particles on the sieve and particles below the sieve (particles passing through the sieve) were mixed at a mass ratio (above sieve/below sieve) of 54/46. As a result, polyethylene oxide particles (d-1) containing 54 mass% of polyethylene oxide particles having a particle size of 300 $\mu$m or more were obtained.

Comparative Example 2

Polyethylene oxide particles 2 obtained in Production Example 3 were classified by a sieve with a mesh opening of 300 $\mu$m, and particles on the sieve and particles below the sieve (particles passing through the sieve) were mixed at a mass ratio (above sieve/below sieve) of 68/32. As a result, polyethylene oxide particles (d-2) containing 68 mass% of polyethylene oxide particles having a particle size of 300 $\mu$m or more were obtained.

Example 1A

2337 mg of polyethylene oxide particles (D-1) obtained in Example 1, 497 mg of acetaminophen as a drug, and 6 mg of magnesium stearate as a lubricant were uniformly mixed to prepare a drug layer component. On the other hand, polyethylene oxide particles 1 obtained in Production Example 2 were classified by a sieve with a mesh opening of 300 $\mu$m, and particles on the sieve and particles below the sieve (particles passing through the sieve) were mixed at a mass ratio (above sieve/below sieve) of 68/32, thereby obtaining polyethylene oxide particles (P-1) containing 68 mass% of polyethylene oxide particles having a particle size of 300 $\mu$m or more. 988 mg of polyethylene oxide particles (P-1), 415 mg of sodium chloride as an osmotic agent, 14 mg of iron sesquioxide as a colorant, and 3 mg of magnesium stearate as a lubricant were uniformly mixed to prepare a pump layer component.

Then, 284 mg of the drug layer component was placed in a tableting mortar with a diameter of 10 mm and compressed with a compression force of 200 N to form a drug layer, and 142 mg of the pump layer component was compressed and molded thereon with a compression force of 5000 N to laminate a pump layer, thereby forming a core part having a diameter of 10 mm and a thickness of 5.5 to 6.5 mm. 0.9 g of cellulose acetate and 0.1 g of polyethylene glycol 4000 as a plasticizer were dissolved in 100 mL of acetone, and the core part was spray-coated with the resulting solution, thereby forming 52 mg of a coating layer on the surface of the core part. After drying, an opening with a diameter of 1 mm was formed in the coating layer (on the semipermeable membrane) at the center of the drug layer side using an electric drill, thereby obtaining a controlled drug release preparation in the form shown in Fig. 1.

Example 2A

A controlled drug release preparation was obtained in the same manner as in Example 1A, except that polyethylene oxide particles (D-2) obtained in Example 2 were used in place of polyethylene oxide particles (D-1).

Example 3A

A controlled drug release preparation was obtained in the same manner as in Example 1A, except that polyethylene oxide particles (D-3) obtained in Example 3 were used in place of polyethylene oxide particles (D-1).

Comparative Example 1A

A controlled drug release preparation was obtained in the same manner as in Example 1A, except that polyethylene oxide particles (d-1) obtained in Comparative Example 1 were used in place of polyethylene oxide particles (D-1).

Comparative Example 2A

A controlled drug release preparation was obtained in the same manner as in Example 1A, except that polyethylene oxide particles (d-2) obtained in Comparative Example 2 were used in place of polyethylene oxide particles (D-1).

Example 4A

A controlled drug release preparation was obtained in the same manner as in Example 1A, except that polyethylene oxide particles (P-2) were used in place of polyethylene oxide particles (P-1). Polyethylene oxide particles (P-2) were produced in the following manner. Polyethylene oxide particles 1 obtained in Production Example 2 were classified by a sieve with a mesh opening of 300 μm, and particles on the sieve and particles below the sieve (particles passing through the sieve) were mixed at a mass ratio (above sieve/below sieve) of 32/68, thereby obtaining polyethylene oxide particles (P-2) containing 32 mass% of polyethylene oxide particles having a particle size of 300 μm or more.

Example 5A

A controlled drug release preparation was obtained in the same manner as in Example 4A, except that polyethylene oxide particles (D-2) obtained in Example 2 were used in place of polyethylene oxide particles (D-1).

Example 6A

A controlled drug release preparation was obtained in the same manner as in Example 4A, except that polyethylene oxide particles (D-3) obtained in Example 3 were used in place of polyethylene oxide particles (D-1).

Comparative Example 3A

A controlled drug release preparation was obtained in the same manner as in Example 4A, except that polyethylene oxide particles (d-1) obtained in Comparative Example 1 were used in place of polyethylene oxide particles (D-1).

Comparative Example 4A

A controlled drug release preparation was obtained in the same manner as in Example 4A, except that polyethylene oxide particles (d-2) obtained in Comparative Example 2 were used in place of polyethylene oxide particles (D-1).

Test Example

For the preparations obtained in the Examples and Comparative Examples, a dissolution test was performed according to the Japanese Pharmacopoeia (paddle method). In the dissolution test, the test liquid was ion-exchange water, the test temperature was 37.0°C, and the stirring speed was 100 r/min.

Test Results

Tables 1 and 2 show the test results of the preparations obtained in the Examples and Comparative Examples. Specifically, the tables show the results of measuring the drug dissolution rate every hour (1 hour to 24 hours) from the start of the test to each predetermined time, the time for which the dissolution rate exceeded 5%, the time for which the dissolution rate exceeded 10%, and the dissolution speed until the dissolution rate reached from 5% to 80% (expressed as "Dissolution speed at dissolution rate from 5 to 80%" in the tables). The dissolution speed until the dissolution rate reached from 5% to 80% (average dissolution rate) was determined in the following manner. A regression line was created by recording the dissolution rate every hour from when the dissolution rate reached 5% to when it reached 80%. The slope of the line was taken as the dissolution speed until the dissolution rate reached from 5% to 80% (average dissolution rate).

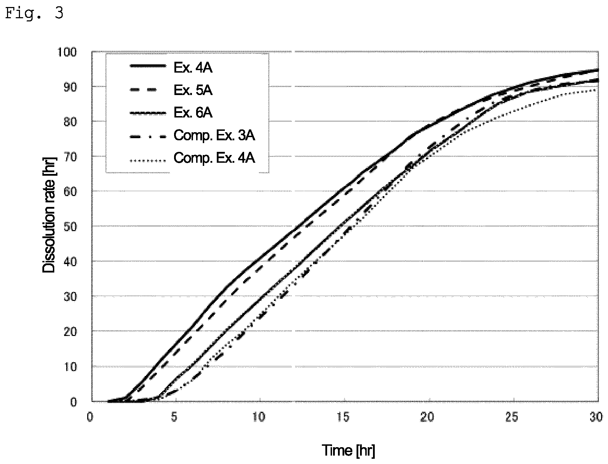

Figs. 2 and 3 show changes over time in the drug dissolution rate of the preparations obtained in the Examples and Comparative Examples from the start of the dissolution test to 30 hours later.

The drug dissolution rate was determined in such a manner that the absorbance (244 nm) of the test liquid T hours after the start of the dissolution test, and the absorbance of the test liquid after the entire drug was released were measured, and the drug dissolution rate was determined by the following formula (1):

Dissolution rate (%) = (absorbance after T hours/absorbance after release of entire drug)×100       (1)

Table 1

| Example/ Comparative Example | 300 μm on (mass%) | | Dissolution rate at each dissolution time (%) | | | | | | | | | | Time for which dissolution rate exceeds X% (hr) | | Dissolution speed at dissolution rate from 5 to 80% (%/hr) |
|---|---|---|---|---|---|---|---|---|---|---|---|---|---|---|---|
| | Drug layer | Pump layer | 1hr | 2hr | 3hr | 4hr | 5hr | 6hr | 7hr | 8hr | 12hr | 24hr | X=5 | X=10 | |
| Example 1A | 14 | 68 | 0.1 | 1.6 | 8.0 | 14.3 | 19.8 | 25.1 | 30.3 | 35.0 | 51.1 | 88.9 | 3 | 4 | 3.71 |
| Example 2A | 2 | 68 | 0.1 | 1.4 | 6.1 | 13.0 | 19.5 | 25.8 | 31.1 | 36.0 | 53.2 | 89.8 | 3 | 4 | 3.71 |
| Example 3A | 32 | 68 | 0.1 | 0.0 | 0.0 | 2.2 | 7.6 | 13.2 | 18.8 | 23.6 | 41.3 | 81.2 | 5 | 6 | 3.89 |
| Comparative Example 1A | 54 | 68 | 0.1 | 0.0 | 0.6 | 0.0 | 2.0 | 5.6 | 9.5 | 14.3 | 28.0 | 78.0 | 6 | 8 | 4.20 |
| Comparative Example 2A | 68 | 68 | 0.1 | 0.0 | 0.5 | 1.4 | 4.5 | 8.4 | 15.9 | 20.6 | 39.1 | 81.4 | 6 | 7 | 4.11 |

Table 2

| Example/ Comparative Example | 300 μm on (mass%) | | Dissolution rate at each dissolution time (%) | | | | | | | | | | Time for which dissolution rate exceeds X% (hr) | | Dissolution speed at dissolution rate from 5 to 80% (%/hr) |
|---|---|---|---|---|---|---|---|---|---|---|---|---|---|---|---|
| | Drug layer | Pump layer | 1hr | 2hr | 3hr | 4hr | 5hr | 6hr | 7hr | 8hr | 12hr | 24hr | X=5 | X=10 | |
| Example 4A | 14 | 32 | 0.1 | 0.1 | 4.0 | 8.9 | 14.0 | 22 | 27.3 | 32.5 | 49.0 | 88.1 | 4 | 5 | 3.81 |
| Example 5A | 2 | 32 | 0.1 | 1.1 | 5.5 | 11.3 | 16.4 | 19.0 | 23.8 | 29.0 | 47.0 | 87.3 | 3 | 4 | 3.99 |
| Example 6A | 32 | 32 | 0.0 | 0.0 | 0.0 | 1.5 | 6.3 | 11 | 15.4 | 20.3 | 38.0 | 84.9 | 5 | 6 | 4.19 |
| Comparative Example 3A | 54 | 32 | 0.0 | 0.2 | 0.6 | 1.3 | 3.2 | 6.4 | 10.6 | 14.6 | 33.3 | 85.8 | 6 | 7 | 4.58 |
| Comparative Example 4A | 68 | 32 | 0.0 | 0.2 | 0.5 | 0.7 | 3.0 | 6.1 | 11.8 | 16.2 | 34.5 | 81.1 | 6 | 7 | 4.14 |

Compared with the preparations obtained in the Comparative Examples, the preparations obtained in the Examples showed a shorter time for the drug dissolution rate to reach 5%, i.e., shorter dissolution start time. Thus, they had the effect of advancing the drug dissolution start (initial drug dissolution), i.e., the effect of shortening the induction period. Further, the comparison of two series of experiments in which the particle size of the pump layer was changed revealed that the effect of shortening the induction period until the start of dissolution was obtained regardless of the particle size of polyethylene oxide particles P contained in the pump layer.

Therefore, it was demonstrated that by applying polyethylene oxide particles D comprising 40 mass% or less of polyethylene oxide particles having a particle size of 300 $\mu$m or more to the drug layer of the controlled drug release preparation, the induction period until the start of drug dissolution could be shortened, and the initial drug dissolution speed could be easily controlled.

Industrial Applicability

The controlled drug release preparation of the present invention can be used for antidepressants, antihypertensive agents, urinary system disease therapeutic agents, anticancer agents, and the like.

Reference Signs List

A.    Controlled drug release preparation
1.    Drug layer
2.    Pump layer
3.    Coating layer
4.    Opening

**Claims**

**1.**    A controlled drug release preparation comprising a controlled drug release preparation composition,

> the controlled drug release preparation having an osmotic pump structure with a core part and a coating layer coating the core part,
> the core part having a drug layer containing a drug, and a pump layer containing an osmotic agent,
> the coating layer being at least partially a semipermeable membrane,
> the coating layer having an opening for releasing the drug to the outside,
> the drug layer comprising a controlled drug release preparation composition,
> the controlled drug release preparation composition comprising polyalkylene oxide particles D,
> the polyalkylene oxide particles D comprising 40 mass% or less of polyalkylene oxide particles having a particle size of 300 $\mu$m or more,
> the pump layer comprising polyalkylene oxide particles P,
> the polyalkylene oxide particles P comprising 80 mass% or less of polyalkylene oxide particles having a particle size of 300 $\mu$m or more,
> wherein the content ratio of polyalkylene oxide particles having a particle size of 300 $\mu$m or more in polyalkylene oxide particles D or P is calculated by classifying polyalkylene oxide particles D or P by a sieve with a mesh opening of 300 $\mu$m according to JIS Z 8801-1, standard sieve, in accordance with the description.

**2.**    The controlled drug release preparation according to claim 1, wherein polyalkylene oxide particles D have a 5 mass% aqueous solution viscosity of 30 to 20000 mPa·s,
wherein the 5 mass% aqueous solution viscosity of polyalkylene oxide particles D is measured by using a rotational viscometer in accordance with the description.

**3.**    The controlled drug release preparation according to claim 1 or 2, wherein polyalkylene oxide particles D have a bulk specific gravity of 0.35 to 0.60 g/mL.

**4.**    The controlled drug release preparation according to any one of claims 1 to 3, wherein silica is contained in an amount of 1.5 parts by mass or less based on 100 parts by mass of the total amount of polyalkylene oxide particles D.

**5.**    The controlled drug release preparation according to any one of claims 1 to 4, wherein polyalkylene oxide particles P have a 0.5 mass% aqueous solution viscosity of 20 to 5000 mPa·s,
wherein the 0.5 mass% aqueous solution viscosity of polyalkylene oxide particles P is measured by using a rotational viscometer in accordance with the description.

**Patentansprüche**

1. Eine Zubereitung zur kontrollierten Wirkstofffreisetzung, umfassend eine Zubereitungszusammensetzung zur kontrollierten Wirkstofffreisetzung,

   wobei die Zubereitung zur kontrollierten Wirkstofffreisetzung eine osmotische Pumpenstruktur mit einem Kernteil und einer den Kernteil beschichtende Beschichtungsschicht aufweist,
   der Kernteil eine einen Wirkstoff enthaltende Wirkstoffschicht und eine ein osmotisches Mittel enthaltende Pumpenschicht aufweist,
   die Beschichtungsschicht zumindest teilweise eine semipermeable Membran ist,
   die Beschichtungsschicht eine Öffnung zum Freisetzen des Wirkstoffs nach außen aufweist,
   die Wirkstoffschicht eine Zubereitungszusammensetzung zur kontrollierten Wirkstofffreisetzung umfasst,
   die Zubereitungszusammensetzung zur kontrollierten Wirkstofffreisetzung Polyalkylenoxidpartikel D umfasst,
   die Polyalkylenoxidpartikel D 40 Massen-% oder weniger Polyalkylenoxidpartikel mit einer Partikelgröße von 300 $\mu$m oder mehr umfassen,
   die Pumpenschicht Polyalkylenoxidpartikel P umfasst,
   die Polyalkylenoxidpartikel P 80 Massen-% oder weniger Polyalkylenoxidpartikel mit einer Partikelgröße von 300 $\mu$m oder mehr umfassen,
   wobei der Gehaltsanteil an Polyalkylenoxidpartikeln mit einer Partikelgröße von 300 $\mu$m oder mehr in den Polyalkylenoxidpartikeln D oder P, durch Klassifizieren der Polyalkylenoxidpartikel D oder P mit einem Sieb mit einer Maschenweite von 300 $\mu$m gemäß JIS Z 8801-1, Standardsieb, gemäß der Beschreibung berechnet wird.

2. Die Zubereitung zur kontrollierten Wirkstofffreisetzung gemäß Anspruch 1, wobei die Polyalkylenoxidpartikel D eine Viskosität in 5-Massen-%iger wässriger Lösung von 30 bis 20000 mPa·s aufweisen,
   wobei die Viskosität in 5-Massen-%iger wässriger Lösung der Polyalkylenoxidpartikel D unter Verwendung eines Rotationsviskosimeters gemäß der Beschreibung gemessen wird.

3. Die Zubereitung zur kontrollierten Wirkstofffreisetzung gemäß Anspruch 1 oder 2, wobei die Polyalkylenoxidpartikel D eine spezifische Schüttdichte von 0,35 bis 0,60 g/ml aufweisen.

4. Die Zubereitung zur kontrollierten Wirkstofffreisetzung gemäß einem der Ansprüche 1 bis 3, wobei Siliciumdioxid in einer Menge von 1,5 Massenteilen oder weniger, bezogen auf 100 Massenteile der Gesamtmenge an Polyalkylenoxidpartikeln D, enthalten ist.

5. Die Zusammensetzung zur kontrollierten Wirkstofffreisetzung gemäß einem der Ansprüche 1 bis 4, wobei die Polyalkylenoxidpartikel P eine Viskosität in 0,5-Massen-%iger wässriger Lösung von 20 bis 5000 mPa·s aufweisen,
   wobei die Viskosität in 0,5-Massen-%iger wässriger Lösung der Polyalkylenoxidpartikel P unter Verwendung eines Rotationsviskosimeters gemäß der Beschreibung gemessen wird.

**Revendications**

1. Préparation pour libération contrôlée de médicament, comprenant une composition de préparation pour libération contrôlée de médicament,

   la préparation pour libération contrôlée de médicament ayant une structure de pompe osmotique avec une partie de cœur et une couche de revêtement qui revêt la partie de cœur,
   la partie de cœur ayant une couche de médicament contenant un médicament, et une couche de pompe contenant un agent osmotique,
   la couche de revêtement étant au moins partiellement une membrane semi-perméable,
   la couche de revêtement ayant une ouverture pour libérer le médicament vers l'extérieur,
   la couche de médicament comprenant une composition de préparation pour libération contrôlée de médicament,
   la composition de préparation pour libération contrôlée de médicament comprenant des particules de poly(oxyde d'alkylène) D,

les particules de poly(oxyde d'alkylène) D comprenant 40 % en masse ou moins de particules de poly(oxyde d'alkylène) ayant une taille de particule de 300 µm ou plus,

la couche de pompe comprenant des particules de poly(oxyde d'alkylène) P,

les particules de poly(oxyde d'alkylène) P comprenant 80 % en masse ou moins de particules de poly(oxyde d'alkylène) ayant une taille de particule de 300 µm ou plus,

dans laquelle la proportion en contenu de particules de poly(oxyde d'alkylène) ayant une taille de particule de 300 µm ou plus dans les particules de poly(oxyde d'alkylène) D ou P est calculée par classification des particules de poly(oxyde d'alkylène) D ou P au moyen d'un tamis ayant une ouverture de maille de 300 µm, conformément à la norme JIS Z 8801-1, tamis normalisé, conformément à la description.

2. Préparation pour libération contrôlée de médicament selon la revendication 1,

dans laquelle les particules de poly(oxyde d'alkylène) D ont une viscosité en solution aqueuse à 5 % en masse de 30 à 20 000 mPa·s,

dans laquelle la viscosité en solution aqueuse à 5 % en masse des particules de poly(oxyde d'alkylène) D est mesurée par utilisation d'un viscosimètre rotatif conformément à la description.

3. Préparation pour libération contrôlée de médicament selon la revendication 1 ou 2, dans laquelle les particules de poly(oxyde d'alkylène) D ont une masse volumique apparente de 0,35 à 0,60 g/ml.

4. Préparation pour libération contrôlée de médicament selon l'une quelconque des revendications 1 à 3, dans laquelle de la silice est contenue en une quantité de 1,5 parties en masse ou moins pour 100 parties en masse de la quantité totale des particules de poly(oxyde d'alkylène) D.

5. Préparation pour libération contrôlée de médicament selon l'une quelconque des revendications 1 à 4, dans laquelle les particules de poly(oxyde d'alkylène) P ont une viscosité en solution aqueuse à 0,5 % en masse de 20 à 5 000 mPa·s,

dans laquelle la viscosité en solution aqueuse à 0,5 % en masse des particules de poly(oxyde d'alkylène) P est mesurée par utilisation d'un viscosimètre rotatif conformément à la description.

Fig. 1

Fig. 2

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S6041609 A
- JP S61103820 A
- JP H06505263 A
- WO 9737640 A2